# EUROPEAN PATENT APPLICATION

(11) **EP 1 762 234 A1**
(43) Date of publication of application: **14.03.2007**
(21) Application number: 05753416.6
(22) Date of filing: 21.06.2005
(51) Int. Cl.: A61K 31/05, A23L 1/30, A23L 2/52, A61K 36/18, A61P 3/04, A61P 3/06, A61P 3/10, A61P 43/00

(54) **AMPK ACTIVATOR**

(30) Priority: 28.06.2004 JP 2004189150; 04.03.2005 JP 2005059874
(71) Applicant: KAO CORPORATION, Tokyo 103-8210 (JP)
(72) Inventor: MURASE, T. Kao Corp. Research Lab., Haga-gun Tochigi 3213497 (JP)
(74) Representative: Vossius & Partner
(86) International application number: PCT/JP2005/011371
(87) International publication number: WO 2006/001278

(57) **Abstract**

The present invention relates to an AMPK activator containing resveratrol as an active ingredient.

The present invention provides an AMPK activator containing, as an active ingredient, an ingredient contained in a naturally occurring material which has been consumed as a food or beverage for a long period of time, which exhibits high safety, which is readily available, which exhibits excellent processability, and which can be employed practically in humans.

## Description

### Field of the Invention

The present invention relates to an AMPK (AMP-activated protein kinase) activator.

### Background of the Invention

Obesity is caused by an excess of ingested energy compared with consumed energy. Therefore, it is crucially important to lessen ingested energy (i.e., less eating) or instead heighten consumed energy by exercise, in order to prevent and ameliorate obesity. In the modernized society, the growing trend toward the westernization of eating habits has brought about a sharp increase of fat intake, and the massive wave of motorization has cut the time needed for exercise as well, thus leading to less consumption of energy. As a result, the number of obese people is on the rise, and lifestyle-related diseases (e.g., diabetes) has become a serious social problem at the same time. Exercise is widely known to promote energy metabolism, so it is an effective measure to prevent or ameliorate a variety of lifestyle-related diseases, including obesity and diabetes. Nonetheless, it is too difficult to take exercise routinely in real life. If, however, there is any other measure that may be able to bring about the same effect as that obtained from exercise, such a measure would become a "exercise-substitutive means" that can be expected to have an equivalent effect without actual exercise. Such a substitutive means is also expected to work more effectively even with limited exercise.
Hitherto, various attempts have been made to prevent or ameliorate obesity and diabetes. There have been disclosed a variety of compounds and extracts which are considered effective for preventing or ameliorating obesity, including soybean extract (Patent Document 1), cyanidin 3-glucoside (Patent Document 2), sugarcane polyphenol (Patent Document 3), D-cysteinolic acid (Patent Document 4), and conjugated-trienoic-acid-containing oil and fat (Patent Document 5). Reality, however, is that these compounds' utilities remain to be sufficiently elucidated in terms of their action mechanism and safety, because so far there has not been enough data about the eating performance thereof. To our knowledge, little is known about methods or food materials which can substitute for the function of exercise or enhance the effects of physical exercises.

In the meantime, studies on energy metabolism, obesity, and the mechanism of diabetes development have made much progress so far, and it has thus become apparent that AMPK plays a crucial role in such events (Non-Patent Document 1). AMPK functions as a "metabolic sensor," which increases its activity under the conditions where the intracellular ATP level is reduced, and promotes ATP synthesis through metabolic stimulation. Activation of AMPK in muscles, which requires a large amount of energy during exercise, is known to play a great role in producing energy during exercise. Recent studies suggests that AMPK is activated by, for example, an adipocyte-derived hormone (Non-Patent Document 3) (e.g., leptin (Non-Patent Document 2) or adiponectin) or metformin (i.e., a diabetes treatment drug) (Non-Patent Document 4), and AMPK serves as an intracellular mediator for the promotion of glucose utilization or fatty acid oxidation induced by such a compound. For example, AMPK is known to affect fatty acid oxidation in mitochondria through control of the activity of acetyl-CoA carboxylase (ACC). Carnitine palmitoyltransferase (CPT-1), which incorporates a long-chain fatty acid into mitochondria, is an enzyme that determines the rate of fatty acid oxidation in mitochondria, and is strongly inhibited by malonyl CoA, which is produced by ACC. Therefore, AMPK is thought to suppress the activity of ACC by transforming ACC into a phosphorylating form (Ser 79) and also reduce the amount of malonyl CoA, thereby accelerating fatty acid oxidation as the activity of CPT-1 is enhanced.
Patent Document 1: JP-A-2003-286180
Patent Document 2: JP-A-2003-252766
Patent Document 3: JP-A-2003-137803
Patent Document 4: JP-A-2003-104879
Patent Document 5: JP-A-2002-186424
Non-Patent Document 1: Molecular Medicine, Vol. 39, No. 4, pp. 398-407, 2002
Non-Patent Document 2: Nature, Vol. 415, pp. 339-343, 2002
Non-Patent Document 3: Nature, Vol. 423, pp. 762-769, 2003
Non-Patent Document 4: J. Clin. Invest., Vol. 108, pp. 1167-1174, 2001

### Disclosure of the Invention

The present invention provides an AMPK activator containing resveratrol as an active ingredient.

### Modes for Carrying Out the Invention

As described above, AMPK is activated under the conditions where intracellular energy is insufficient, and plays an important role in energy metabolism or nutrient metabolism in vivo. Specifically, activation of AMPK is considered to contribute to the promotion of energy expenditure, and prevention or amelioration of obesity, diabetes, insulin resistance, or hyperlipemia. An AMPK activator is envisaged to exhibit effects similar to those of exercise, and thus is considered an effective agent which functions as a substitute for physical exercise (hereinafter the agent may be referred to as an "exercise-substitutive agent").

Conventionally known AMPK-activating compounds include, in addition to the aforementioned leptin, adiponectin, and metformin, AICAR (5-aminoimidazole-4-carboxamide). However, at present, there is barely known a material which has been consumed as a food or beverage ingredient for a long period of time, which exhibits high safety, which is readily available, which exhibits excellent processability, and which can be employed practically in humans.

The present invention provides an AMPK activator containing, as an active ingredient, an ingredient contained in a naturally occurring material which has been consumed as a food or beverage for a long period of time, which exhibits high safety, which is readily available, which exhibits excellent processability, and which can be employed practically in humans.
The present inventors have searched for ingredients that are effective for activation of AMPK among naturally occurring materials which have been consumed as a food or beverage for a long period of time, and have found that resveratrol, which has been consumed as a food or beverage ingredient for a long period of time through intake of grapes or processed grape products (e.g., wine), exhibits the effect of AMPK activation, which is effective for promoting energy metabolism or lipid metabolism, thereby preventing or ameliorating obesity, diabetes, hyperglycemia, hepatic hypertrophy, fatty liver, hypercholesterolemia, insulin resistance, or hyperlipemia.

The AMPK activator of the present invention contains an ingredient which has been consumed as a food or beverage ingredient for a long period of time, exhibits excellent safety, is excellent in AMPK activation in liver cells and muscle cells, induces activation of energy metabolism (e.g., lipid metabolism or glucose metabolism), and is effective for preventing or ameliorating obesity, diabetes, hyperglycemia, insulin resistance, hypercholesterolemia, hepatic hypertrophy, or fatty liver. A food containing the AMPK activator of the present invention could exhibit an exercise-substitutive effect.

As used herein, the term "resveratrol" collectively refers to trans-resveratrol, cis-resveratrol, and a mixture thereof.
Resveratrol, which is a type of polyphenol, is found in, for example, the leaves and skins of grapes, and has been reported to exhibit an LDL oxidation inhibitory effect, platelet aggregation inhibitory effect, anti-inflammatory effect, anticancer effect, etc. ("Roka Yobo Shokuhin no Kaihatsu" ("Development of Anti-Aging Foods"), CMC Publishing Co., Ltd., pp. 156-168, 1999). In addition, resveratrol has been known to be useful as, for example, an antithrombotic agent (JP-A-61-171427), a drug for inhibiting progression of circulatory disorder (JP-A-2003-300904), a preventive or therapeutic agent for hyperlipemia (JP-A-2001-72583), or a preventive or therapeutic agent for bone diseases (JP-A-2000-281567).

Resveratrol to be employed may be obtained in the form of a highly pure product through organic chemical synthesis or synthesis employing a microorganism, or may be extracted from a resveratrol-containing naturally occurring substance. In the latter case, the resultant extract per se may be employed, or resveratrol isolated from the extract may be employed. No particular limitation is imposed on the naturally occurring substance, so long as it contains resveratrol. Examples of the naturally occurring substance include grape, earthnut, peanut, and polygonaceous plants such as *Polygonum cuspidatum* and *Polygonum multiflorum.*

The naturally occurring substance is particularly preferably a grape. No particular limitation is imposed on the type of grape, but preferred are Delaware, Kyoho, Koshu, Pione, Muscat, Chenin Blanc, Grenache, Mataro, Muller-Thurgau, Trebbiano, Berry A, Cabernet Sauvignon, Merlot, Pinot Noir, Cabernet Franc, Syrah, Chardonnay, Sauvignon Blanc, Semillon, Shiraz, Gamay, Riesling, Aligote, etc. As used herein, the term "grape extract" refers to an extract from the fruit or leaf of a grape, or from a grape-derived product. Examples of the grape-derived product include grape juice, wine, residues obtained during wine production, and wine concentrates. Resveratrol is extracted from, for example, the skin or leaf of a grape, which contains a large amount of resveratrol. Resveratrol extraction is carried out through a generally employed extraction technique by use of, for example, water, hot water, aqueous alcohol, or an organic solvent. Wine, a wine concentrate, or a solid obtained through concentration to dryness of wine, which contains resveratrol, may be employed for extraction. Grape residues obtained during wine production may be employed as they are, or the residues may be subjected to extraction with, for example, an organic solvent/water. The organic solvent to be employed is preferably an alcohol (e.g., ethanol), particularly preferably ethanol.

The AMPK activator of the present invention may be administered in the form of a drug to a human or an animal. Alternatively, the AMPK activator may be incorporated into a variety of foods and beverages or pet foods so as to be consumed by humans or animals. The AMPK activator may be applied to a common food or beverage; or may be applied to a functional food or beverage, a food for a subject suffering a disease, or a food for specified health use, the food (or beverage) bearing a label thereon indicating that it has a physiological function; for example, prevention or amelioration of obesity, body fat accumulation, diabetes, fatty liver, a lifestyle-related disease, lipid metabolism promotion, hyperglycemia, insulin resistance, or hypercholesterolemia. The AMPK activator of the present invention activates AMPK, and ameliorates the aforementioned various symptoms, which are caused by, for example, lack of exercise. Therefore, the AMPK activator is useful as an exercise-substitutive agent exhibiting effects similar to those of exercise, particularly as an exercise-substitutive agent for preventing or ameliorating obesity, body fat accumulation, diabetes, fatty liver, a lifestyle-related disease, etc. The AMPK activator may be formulated into a drug product; for example, a peroral solid product such as a tablet or a granule, or a peroral liquid product such as a solution or a syrup. The AMPK activator is useful as a drug product such as a lipid metabolism activating agent, an obesity suppressing agent, a diabetes preventing/ameliorating agent, a hepatic hypertrophy suppressing agent, a fatty liver suppressing agent, or a lifestyle-related disease preventing/ameliorating agent.

The amount of resveratrol to be incorporated into such a product varies depending on the form of the product. When resveratrol is incorporated into, for example, a food or beverage or a pet food, the incorporation amount is generally 0.0001 to 5 mass%, preferably 0.001 to 5 mass%, more preferably 0.01 to 1 mass%. When resveratrol is incorporated into a drug product other than the aforementioned products, such as a peroral solid product (e.g., a tablet, a granule, or a capsule) or a peroral liquid product (e.g., a solution or a syrup), the incorporation amount is generally 0.01 to 95 mass%, preferably 5 to 95 mass%, more preferably 20 to 90 mass%.

The daily dose (effective intake) of the AMPK activator of the present invention is preferably 5 to 2,000 mg/60 kg body weight, more preferably 10 to 1,000 mg/60 kg body weight, even more preferably 50 to 500 mg/60 kg body weight.

### Examples

### Example 1

The AMPK activation effect of resveratrol was evaluated on the basis of phosphorylation of AMPK α and β through the below-described method by use of a mouse hepatocyte line (Hepa 1-6).

Mouse hepatocyte cells (Hepa 1-6) were seeded in a 25-cm² flask, and cultured in DMEM (+ 10% FBS, + antibacterial agent) at 37°C for one to two days. The culture medium was removed when the cells became subconfluent, and the cells were washed with PBS (-), followed by replacement with DMEM (- FBS) and further culturing for one day. After the culture medium was removed, DMEM (- FBS) containing a predetermined amount of resveratrol was added, followed by culturing for 60 minutes. Thereafter, the culture medium was removed, and the cells were washed with PBS (-), followed by addition of 200 µL of a cytolytic solution (10 mmol/L Tris (pH 7.4), 50 mmol/L sodium chloride, 30 mmol/L sodium pyrophosphate, 0.5 mass% Triton X-100, protease inhibitor cocktail (SIGMA P2714), phosphatase inhibitor cocktail-2 (SIGMA P5726)). The resultant cell lysate was collected with a cell scraper. The thus-collected cell lysate was thoroughly homogenized by passing it through a syringe using a 23-G needle (3 times), and then the cell lysate was allowed to stand on ice for 30 minutes. The cell lysate was subjected to centrifugation at 15,000 r/min and 4°C for 15 minutes. Thereafter, the resultant supernatant protein was employed for the below-described measurement.

After the concentration of the supernatant protein was measured, the protein concentration was regulated to a constant level between samples. After addition of SDS buffer (250 mmol/L Tris, 12.5 mass% SDS, 20 mass% glycerin) in an amount 1/4 that of the supernatant protein, 2-mercaptoethanol and bromophenol blue were further added, followed by thermal denaturation at 95°C and quenching at 4°C, to thereby prepare a sample for electrophoresis.

A predetermined amount (about 20 to about 40 µg) of the electrophoresis sample was subjected to SDS-PAGE (12% gel), followed by transfer to a membrane. Thereafter, phospho-AMPK was detected by use of anti-phospho-AMPKα (Thr72) antibody (product of Cell Signaling) or anti-phospho-AMPKβ (Ser108) antibody (product of Cell Signaling) serving as a primary antibody, anti-rabbit-HRP antibody (product of Amersham) serving as a secondary antibody, and phototope-HRP Western Detection System (product of Cell Signaling) serving as a detection reagent. The intensity of the thus-detected band was converted into numerical data (pixels) through image analysis (EDAS 290 image analysis system: KODAK), and the degree of AMPK activation was calculated relative to the control (i.e., group containing no sample), which was taken as 100. In Example 1, resveratrol (3,4',5-trihydroxy-trans-stilbene) manufactured by SIGMA was employed.

**[Table 1]**

| | Control | Resveratrol (µmol/L) |
|---|---|---|
| | | 150 |
| Phospho-AMPKα | 100 | 538 |
| Phospho-AMPKβ | 100 | 168 |

As is clear from Table 1, resveratrol exhibits a potent AMPK activation effect.

### Example 2

The AMPK activation effect of resveratrol was evaluated on the basis of phosphorylation of AMPK α and β through the below-described method by use of a mouse muscle cell line (C2C12).

Mouse muscle cells (C2C12) were seeded in a 25-cm² flask, and cultured in DMEM (+ 10% FBS, + antibacterial agent) at 37°C for one to two days. The culture medium was removed when the cells reached confluent, and the cells were washed with PBS (-), followed by replacement with DMEM (2 mass% Horse serum) and further culturing for seven to eight days while the culture medium was exchanged every two or three days. Thereafter, the culture medium was removed, and the cells were washed with PBS (-), followed by replacement with DMEM (- FBS) and further culturing for one day. After the culture medium was removed, DMEM (- FBS) containing a predetermined amount of resveratrol was added, followed by culturing for 60 minutes. Subsequently, the culture medium was removed, and the cells were washed with PBS (-), followed by addition of 200 µL of a cytolytic solution (10 mmol/L Tris (pH 7.4.), 50 mmol/L sodium chloride, 30 mmol/L sodium pyrophosphate, 0.5 mass% Triton X-100, protease inhibitor cocktail (SIGMA P2714), phosphatase inhibitor cocktail-1 (SIGMA P2850), phosphatase inhibitor cocktail-2 (SIGMA P5726)). The resultant cell lysate was collected with a cell scraper. The thus-collected cell lysate was thoroughly homogenized by passing it through a syringe using a 23-G needle (3 times), and then the cell lysate was allowed to stand on ice for 30 minutes. The cell lysate was subjected to centrifugation at 15,000 r/min and 4°C for 15 minutes. Thereafter, the resultant supernatant protein was employed for the below-described measurement.

After the concentration of the supernatant protein was measured, the protein concentration was regulated to a constant level between samples. After addition of an SDS buffer (250 mmol/L Tris, 12.5 mass% SDS, 20 mass% glycerin) in an amount 1/4 that of the supernatant protein, 2-mercaptoethanol and bromophenol blue were further added, followed by thermal denaturation at 95°C and quenching at 4°C, to thereby prepare a sample for electrophoresis.

A predetermined amount (about 20 to about 40 µg) of the electrophoresis sample was subjected to SDS-PAGE (12% gel), followed by transfer to a membrane. Thereafter, phospho-AMPK was detected by use of anti-phospho-AMPKα (Thr72) antibody (product of Cell Signaling) or anti-phospho-AMPKβ (Ser108) antibody (product of Cell Signaling) serving as a primary antibody, anti-rabbit-HRP antibody (product of Amersham) serving as a secondary antibody, and phototope-HRP Western. Detection System (product of Cell Signaling) serving as a detection reagent. The intensity of the thus-detected band was converted into numerical data (pixels) through image analysis (EDAS 290 image analysis system: KODAK), and the degree of AMPK activation was calculated relative to the control (i.e., group containing no sample), which was taken as 100. In Example 2, resveratrol manufactured by SIGMA was employed.

**[Table 2]**

| | Control | Resveratrol (µmol/L) |
|---|---|---|
| | | 150 |
| Phospho-AMPKα | 100 | 848 |
| Phospho-AMPKβ | 100 | 425 |

As is clear from Table 2, resveratrol exhibits potent AMPK activation effect in muscle cells.

### Example 3

Efficacy of resveratrol was evaluated as described below.
Seven-week-old C57BL/6 male mice (CLEA Japan, Inc.) were employed, and the animals were divided into three groups, each containing 10 to 15 animals. The animals were fed with diets formulated as shown in Table 3. Twenty-four weeks after initiation of the test, body weight was measured, and blood was collected under anesthesia and non-fasting conditions for measurement of plasma glucose, cholesterol, insulin, and leptin. In addition, visceral fat weight (epididymal fat, retroperitoneal fat, and perirenal fat) and liver weight were measured. In Example 3, resveratrol manufactured by Cayman was employed.

**[Table 3]**

| | Group 1 | Group 2 | Group 3 |
|---|---|---|---|
| Vegetable oil (%) | 5 | 25 | 25 |
| Lard (%) | 0 | 5 | 5 |
| Sucrose (%) | 0 | 13 | 13 |
| Casein (%) | 20 | 20 | 20 |
| Potato starch (%) | 66.5 | 28.5 | 28.3 |
| Vitamin (%) | 1 | 1 | 1 |
| Mineral (%) | 3.5 | 3.5 | 3.5 |
| Cellulose (%) | 4 | 4 | 4 |
| Resveratrol (%) | 0 | 0 | 0.2 |

The results are shown in Table 4. In group 2, a significant increase in body weight is observed as compared with the case of group 1 (P < 0.001). In contrast, in group 3, an increase in body weight is suppressed as compared with the case of group 1, and body weight is significantly lower than that in group 2 (P < 0.001). In group 2, visceral fat weight is significantly higher than that in group 1 (P < 0.001), and in group 3, visceral fat weight is significantly lower than that in group 2 (P < 0.01). As is clear from the test results, resveratrol employed in the present invention (hereinafter may be referred to as "resveratrol of the present invention") exhibits excellent anti-obesity effect and body fat accumulation suppressing effect.
In group 2, a significant increase in liver weight is observed as compared with the case of group 1 (P < 0.01). In contrast, in group 3, an increase in liver weight is suppressed as compared with the case of group 1, and liver weight is significantly lower than that in group 2 (P < 0.05). As is clear from the test results, resveratrol of the present invention exhibits an excellent hepatic hypertrophy suppressing effect. Resveratrol, which suppresses hepatic hypertrophy that would otherwise be caused by the intake of high-fat foods, is considered effective for suppression of fatty liver associated with obesity.

**[Table 4]**

| | Group 1 | Group 2 | Group 3 |
|---|---|---|---|
| Body weight (g) | 32.2 | 38.8 | 32.1 |
| Visceral fat weight (g) | 0.88 | 2.17 | 1.00 |
| Liver weight (g) | 1.34 | 1.62 | 1.39 |

Table 5 shows the results of blood analysis. In group 2, a significant increase in blood glucose level (glucose) is observed as compared with the case of group 1 (P < 0.001). In group 3, blood glucose level is lower than that in group 2 (P < 0.1). Resveratrol of the present invention exhibits an excellent effect of suppressing an increase in blood glucose level and thus is considered effective for diabetes.
In group 2, the total cholesterol level is significantly higher than that in group 1 (P < 0.05). In contrast, in group 3, an increase in total cholesterol level is not observed, and the total cholesterol level is significantly lower than that in group 2 (P < 0.05). As is clear from the test results, resveratrol exhibits an excellent cholesterol suppressing effect.
In group 2, the blood insulin level is higher than that in group 1. In contrast, in group 3, an increase in blood insulin level is lower as compared with the case of group 1, and blood insulin level is lower than that in group 2. Resveratrol of the present invention exhibits an excellent effect of suppressing an increase in blood insulin level, and thus is considered effective for the suppression of insulin resistance.
In group 2, the blood leptin level is significantly higher than that in group 1 (P < 0.01). In contrast, in group 3, an increase in blood leptin level is suppressed as compared with the case of group 1, and blood leptin level is significantly lower than that in group 2 (P < 0.01). Resveratrol of the present invention exhibits an excellent effect of suppressing an increase in blood leptin level, and thus is considered effective for suppression of leptin resistance.

**[Table 5]**

| | Group 1 | Group 2 | Group 3 |
|---|---|---|---|
| Total cholesterol (mg/dL) | 58 | 76 | 60 |
| Glucose (mg/dL) | 133 | 191 | 162 |
| Insulin (ng/mL) | 2.52 | 3.38 | 2.03 |
| Leptin (ng/mL) | 1.43 | 6.19 | 1.79 |

### Example 4: Lipid metabolism activating effect

Seven-week-old Balb/c male mice were divided into two groups, and saline (control) or resveratrol (200 mg/kg body weight) was orally administered for 10 consecutive days. Thereafter, the liver and skeletal muscle (gastrocnemius muscle + soleus muscle) were collected from each of the animals. Each of the thus-collected liver and skeletal muscle was homogenized in buffer (250 mM sucrose, 1 mM EDTA in 10 mM HEPES (pH 7.2)), and insoluble tissue residues were centrifugally separated, whereby a supernatant was obtained. The thus-obtained supernatant was subjected to measurement of protein content, and the protein content was regulated to a constant level between samples. Each of the resultant samples was employed for measurement of lipid metabolism activity (β-oxidation activity). The supernatant protein (100 µg) was reacted with 0.1 µCi [¹⁴C]-palmitic acid at 37°C for 20 minutes in 200 µL (final volume) of buffer (50 mM Tris-HCl (pH 8.0), 40 mM NaCl, 2 mM KCl, 2 mM MgCl₂, 1 mM DTT, 5 mM ATP, 0.2 mM L-carnitine, 0.2 mM NAD, 0.06 mM FAD, 0.12 mM CoA, 3 mM α-cyclodextrin). The reaction was stopped with 200 µL of 0.6 N perchloric acid, and then unreacted [¹⁹C]-palmitic acid was removed three times with hexane (1 mL). Lipid degradation activity was evaluated through measurement of the radioactivity of the aqueous layer.
The measurement results are shown in Table 6. Lipid degradation activity was calculated relative to that of the control, which was taken as 100. Intake of resveratrol considerably increases the lipid degradation activity (β-oxidation activity) of the liver and skeletal muscle (liver: p < 0.001, muscle: p < 0.001). As is clear from the test results, resveratrol is effective for activation of lipid metabolism.
As is clear from the above-described results, resveratrol of the present invention exhibits an AMPK activation effect, induces activation of energy metabolism (e.g., lipid metabolism), and is effective for preventing or ameliorating obesity, diabetes, hyperglycemia, insulin resistance, hypercholesterolemia, hepatic hypertrophy, or fatty liver. In addition, resveratrol of the present invention ameliorates, through its AMPK activation effect, the aforementioned various symptoms, which are caused by, for example, lack of exercise, and therefore is useful as an exercise-substitutive agent exhibiting effects similar to those of exercise, particularly as an exercise-substitutive agent for preventing or ameliorating obesity, body fat accumulation, diabetes, fatty liver, a lifestyle-related disease, etc.

**[Table 6]**

| | Control | Resveratrol |
|---|---|---|
| Liver | 100 | 323 |
| Skeletal muscle | 100 | 243 |

Typical examples of the present invention will next be described, but the invention is not limited to these examples.

### Example 5

### Capsule for preventing or ameliorating lifestyle-related disease

The following composition (300 mg) was encapsulated into a capsule.

| | |
|---|---|
| Resveratrol | 68 mass% |
| Cornstarch | 10 |
| Cellulose | 10 |
| Tocopherol | 2 |
| Lactose | 10 |

### Example 6

### Tablet for preventing or ameliorating lifestyle-related disease

The following composition was subjected to tableting for production of tablets (250 mg per tablet).

| | |
|---|---|
| Grape extract | 50 mass% |
| Cornstarch | 10 |
| Cellulose | 10 |
| Vitamin C | 20 |
| Lactose | 10 |

### Example 7

### Tablet for preventing or ameliorating lifestyle-related disease

The following composition was subjected to tableting for production of tablets (250 mg per tablet).

| | |
|---|---|
| Resveratrol | 50 mass% |
| Cornstarch | 10 |
| Cellulose | 10 |
| Vitamin C | 20 |
| Lactose | 10 |

### Example 8

### Granule for preventing or ameliorating lifestyle-related disease

The following ingredients were mixed for production of a granule (500 mg per package).

| | |
|---|---|
| Resveratrol | 25 mass% |
| Dry grape powder | 20 |
| Fructose | 20 |
| Glucose | 20 |
| Powdered skim milk | 10 |
| Caffeine | 5 |

### Example 9

### Beverage for preventing or ameliorating obesity or lifestyle-related disease

The following ingredients were mixed for production of a fruit juice beverage.

| | |
|---|---|
| Resveratrol | 100 mg |
| Grape juice | 500 mL |

### Example 10

### Beverage for preventing or ameliorating lifestyle-related disease

The following ingredients were mixed for production of a fruit juice beverage.

| | |
|---|---|
| Resveratrol | 50 mg |
| Vitamin C | 300 mg |
| Orange juice | 300 mL |
| Water | 200 mL |
| Perfume | some amount |
| Isomerized sugar | 5 g |

### Example 11

### Beverage for preventing or ameliorating obesity or lifestyle-related disease

The following ingredients were mixed for production of a fruit juice beverage.

| | |
|---|---|
| Dry grape leaf powder | 300 mg |
| Resveratrol | 15 mg |
| Vitamin C | 300 mg |
| Grape juice | 300 mL |
| Water | 200 mL |
| Perfume | some amount |
| Glucose | 2 g |

### Example 12

### Beverage for preventing or ameliorating obesity or lifestyle-related disease

The following ingredients were mixed for production of a tea beverage.

| | |
|---|---|
| Resveratrol | 20 mg |
| Tea catechin | 200 mg |
| Vitamin C | 200 mg |
| Green tea | 500 mL |

### Example 13

### Beverage for preventing or ameliorating obesity or lifestyle-related disease

The following ingredients were mixed for production of a fruit juice beverage.

| | |
|---|---|
| Dry red wine concentrate powder | 2,000 mg |
| Grape juice | 250 mL |
| Water | 250 mL |
| Perfume | some amount |

### Example 14

### Beverage for preventing or ameliorating lifestyle-related disease

The following ingredients were mixed for production of a fruit juice beverage.

| | |
|---|---|
| Resveratrol | 10 mg |
| Vitamin C | 500 mg |
| Grapefruit juice | 25 mL |
| Water | 470 mL |
| Perfume | some amount |
| Isomerized sugar | 5 g |

### Example 15

### Exercise-substitutive beverage

A carbonated beverage having the following composition was produced.

| | |
|---|---|
| Resveratrol | 15 mg |
| Vitamin C | 500 mg |
| Carbonated water | 495 mL |
| Lemon juice | 5 mL |
| Perfume | some amount |
| Aspartame | 5 g |

### Example 16

### Exercise-substitutive beverage

A carbonated beverage having the following composition was produced.

| | |
|---|---|
| Resveratrol | 100 mg |
| Vitamin C | 500 mg |
| Carbonated water | 500 mL |
| Lemon juice | 5 mL |
| Perfume | some amount |
| Aspartame | 5 g |

### Example 17

### Exercise-substitutive food

The following composition was subjected to tableting for production of a chewable tablet food (1,000 mg per tablet).

| | |
|---|---|
| Resveratrol | 2.5 mass% |
| Maltose | 11 |
| Lactose | 30 |
| Glucose | 15 |
| Vitamin C | 20 |
| Vitamin E | 1 |
| Cellulose | 10 |
| Xylitol | 10 |
| Perfume | 0.5 |

## Claims

1. An AMPK activator comprising resveratrol as an active ingredient.

2. A lipid metabolism activating agent comprising resveratrol as an active ingredient.

3. An obesity suppressing agent comprising resveratrol as an active ingredient.

4. A diabetes preventing/ameliorating agent comprising resveratrol as an active ingredient.

5. A hepatic hypertrophy suppressing agent comprising resveratrol as an active ingredient.

6. A fatty liver suppressing agent comprising resveratrol as an active ingredient.

7. A food or beverage comprising resveratrol, and exhibiting the effect of preventing or ameliorating obesity and/or body fat accumulation and/or diabetes and/or fatty liver and/or a lifestyle-related disease, wherein the food or beverage is indicated by a label to be used for such effect.

8. A food or beverage comprising resveratrol, and exhibiting the effect of promoting lipid metabolism, wherein the food or beverage is indicated by a label to be used for such effect.

9. An exercise-substitutive agent comprising resveratrol as an active ingredient.

10. A food or beverage **characterized by** comprising resveratrol and exhibiting an exercise-substitutive function, wherein the food or beverage is indicated by a label to be used for such effect.

11. Use of resveratrol for producing an AMPK activator.

12. Use of resveratrol for producing a lipid metabolism activating agent.

13. Use of resveratrol for producing an obesity suppressing agent.

14. Use of resveratrol for producing a diabetes preventing/ameliorating agent.

15. Use of resveratrol for producing a hepatic hypertrophy suppressing agent.

16. Use of resveratrol for producing a fatty liver suppressing agent.

17. Use of resveratrol for producing an exercise-substitutive agent.

18. A method for activating AMPK, **characterized by** comprising administration of an effective amount of resveratrol.

19. A method for activating lipid metabolism, **characterized by** comprising administration of an effective amount of resveratrol.

20. A method for suppressing obesity, **characterized by** comprising administration of an effective amount of resveratrol.

21. A method for preventing or ameliorating diabetes, **characterized by** comprising administration of an effective amount of resveratrol.

22. A method for suppressing hepatic hypertrophy, which comprises administration of an effective amount of resveratrol.

23. A method for suppressing fatty liver, which comprises administration of an effective amount of resveratrol.

24. An exercise-substitutive method comprising administration of an effective amount of resveratrol.
